# EUROPEAN PATENT APPLICATION

(11) **EP 4 552 681 A1**
(43) Date of publication of application: **14.05.2025**
(21) Application number: 22950366.9
(22) Date of filing: 21.10.2022
(51) Int. Cl.: A61M 27/00, A61M 1/00

(54) **DRAINAGE TUBE GUIDE DEVICE**

(30) Priority: 04.07.2022 KR 20220081857
(71) Applicant: JSR Medical Co., Ltd., Daegu 42713 (KR); Kim, Jae Hwang, Daegu 42166 (KR)
(72) Inventor: KIM, Jae Hwang, Daegu 42166 (KR)
(74) Representative: Pfenning, Meinig & Partner mbB
(86) International application number: PCT/KR2022/016171
(87) International publication number: WO 2024/010141

(57) **Abstract**

Provided is a drainage tube guide device comprising: a head part which extends in one direction; a guide tube which has a hollow formed therein and extends downward from the head part; and a peeling part which is in communication with the hollow of the guide tube and peels tissue from the front while being inserted thereinto.

## Description

### [Technical Field]

The present invention relates to a drainage tube guide device.

### [Background Art]

A drainage tube is inserted into the surgical site to observe the drainage of body fluids or bleeding after surgery, and to observe the drainage and bleeding of secretions from wounds, secretions accumulated in the body, and secretions after surgery and procedures.

In this case, the drainage tube is fixed to the location desired by the surgeon. However, the drainage tube often comes off the fixing location within a few days after surgery due to the movement (peristalsis) of the intestines that occupy most of the space in the abdominal cavity. As a result, the original purpose of the drainage tube, which is to drain unnecessary body fluids, blood, or secretions, becomes impossible, and it virtually loses its function. The current method to solve this problem is to fix the drainage tube to the peritoneum in the abdominal cavity using sutures, but the results according to this method are not very different.

Recently, a method has been attempted to move the drainage tube to the desired location through a route outside the abdominal cavity, i.e., outside the peritoneum, rather than inside the abdominal cavity, for easy and effective fixation of the drainage tube, and it has been confirmed that it has an improved effect compared to the previous method where the drainage tube was directly inserted inside the abdominal cavity.

The abdominal cavity is composed of the abdominal wall. The abdominal wall is largely composed of skin, subcutaneous fat tissue plane, muscle plane surrounded by fascia in the front and back, fascia, preperitoneal fat tissue plane, and peritoneum. Since the drainage tube itself is made of soft material, an auxiliary device is needed to move the drainage tube to the desired location through a route outside the peritoneum. Among the abdominal wall layers, the layer that is least damaged and can be easily moved is the preperitoneal fat tissue plane.

The preperitoneal fat tissue plane is a physically weak tissue layer and can be easily peeled off. Therefore, among the possible routes outside the peritoneum, passing through the preperitoneal fat tissue plane is the easiest and safest way to reach a target location.

However, insertion of the drainage tube through the peritoneum is impossible with the current technology, and a need for a device which can guide or assist this purpose has been raised, but there is no device which satisfies this need among the existing drainage tubes and related devices.

### [DISCLOSURE]

### [Technical Problem]

The technical object to be achieved by the present invention is to provide a drainage tube guide device capable of guiding a drainage tube to reach a target point while peeling off the extraperitoneum fat tissue plane without damaging tissues and organs.

### [Technical Solution]

According to an embodiment, a drainage tube guide device is provided. The drainage tube guide device comprises a head part which extends in one direction, a guide tube which has a hollow formed therein and extends downward from the head part, and a peeling part which is in communication with the hollow of the guide tube and peels tissue from a front while being inserted thereinto.

The head part may include an inlet which communicates with the hollow of the guide tube so that a drainage tube is able to be inserted into the guide tube from an upper surface.

The head part may include an identification portion protruding forward.

The head part may be any one of a bar shape extending laterally, a sphere forming a curved surface, and a polyhedron forming a polygon.

A length of the guide tube may be 20 cm or more to 40 cm or less.

The guide tube may have a straight shape.

The peeling part may include a fixing portion which is connected to an end of the guide tube, and a protrusion portion which is formed to protrude in an insertion direction.

The fixing portion may include a hollow formed therein, and an end of the guide tube may be inserted into the hollow of the fixing portion.

The protrusion portion may have a round shape with a non-sharp end.

The peeling part may further include an extraction port which is connected to the hollow of the guide tube and through which a drainage tube inserted into the guide tube is extracted.

The extraction port may have a shape of either a circle or an oval.

The peeling part may further include an inclined portion extending upwardly from the fixing portion.

The inclined portion may be curved at an angle of 25 degrees or more to 55 degrees or less from the fixing portion.

A length of the inclined portion may be 1 cm or more to 5 cm or less.

### [Advantageous Effect]

It is possible to guide the drainage tube to the target point while peeling off the extraperitoneum fat tissue plane without damaging tissues and organs.

Since the insertion and operation of the drainage tube is possible through the extraperitoneal route, drainage treatment is possible for the desired period without the drainage tube being displaced due to the movement of the intestine or physical movement after surgery.

### [Description of Drawings]

FIG. 1 is a perspective view of a drainage tube guide device according to an embodiment.
FIG. 2 is a front view of a drainage tube guide device according to an embodiment.
FIG. 3 is a side view of a drainage tube guide device according to an embodiment.
FIG. 4 and FIG. 5 are drawings for describing a head part of a drainage tube guide device according to an embodiment.
FIG. 6 and FIG. 7 are drawings for describing a peeling part of a drainage tube guide device according to another embodiment.

### DETAILED DESCRIPTION OF THE INVENTION

Hereinafter, with reference to the accompanying drawings, an embodiment of the present invention is described in detail so that a person having ordinary skill in the art to which the present invention pertains can easily practice the present invention. However, embodiments of the present invention may be modified in various different ways and are not limited to some embodiments disclosed herein. In the drawings, in order to clearly describe the present invention, parts that are not related to the description have been omitted. Like reference numerals designate like elements throughout the specification.

Throughout the specification, when a part is said to "comprise" a certain component, this does not mean that other components are excluded, but that other components may be included, unless otherwise specifically stated.

The present invention may have various modifications and various embodiments, but specific embodiments will be described in detail by exemplifying them in the drawings. This is not intended to limit the present invention to specific embodiments, but should be understood to correspond to any one of the modifications, equivalents, or substitutes included in the spirit and technical scope of the present invention for connecting and/or fixing structures extending in different directions.

The terms used in this specification are used only to describe specific embodiments and are not intended to limit the present invention. Singular expressions include plural expressions unless the context clearly indicates otherwise.

In addition, when describing the present invention, if it is judged that a detailed description of a related known technology may unnecessarily obscure the gist of the present invention, the detailed description is omitted.

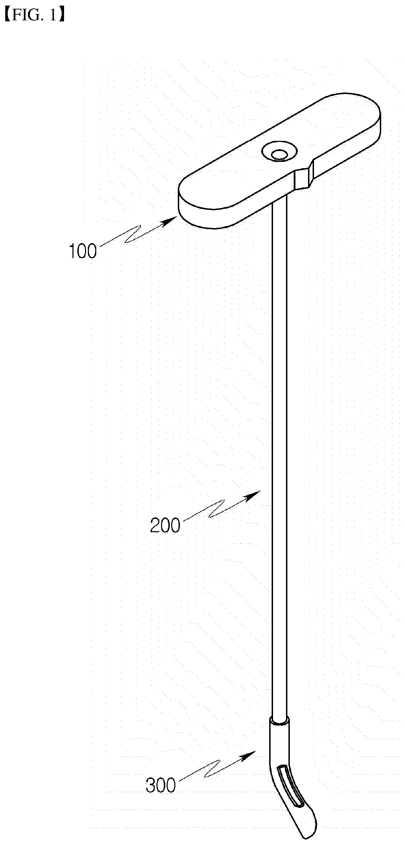

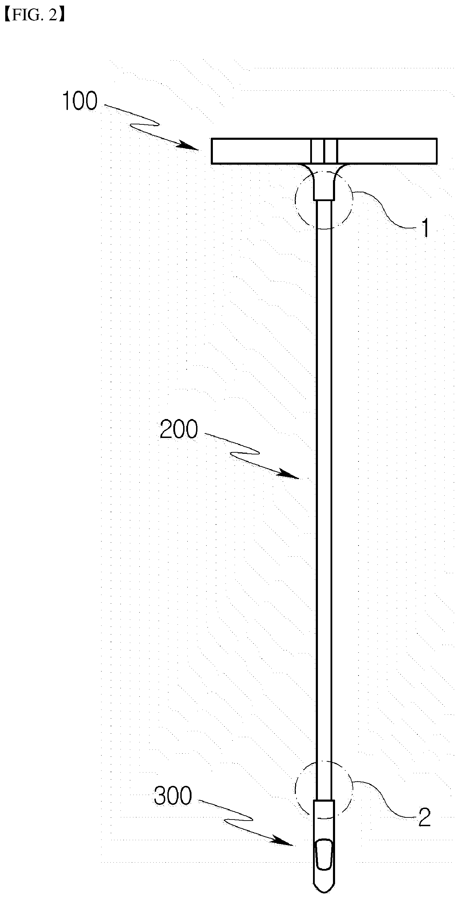

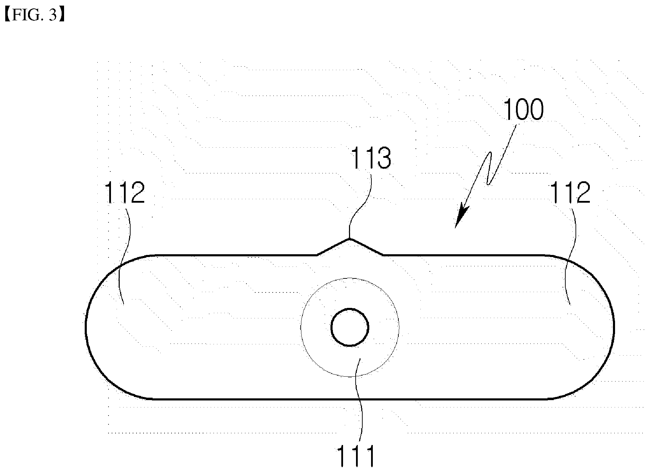

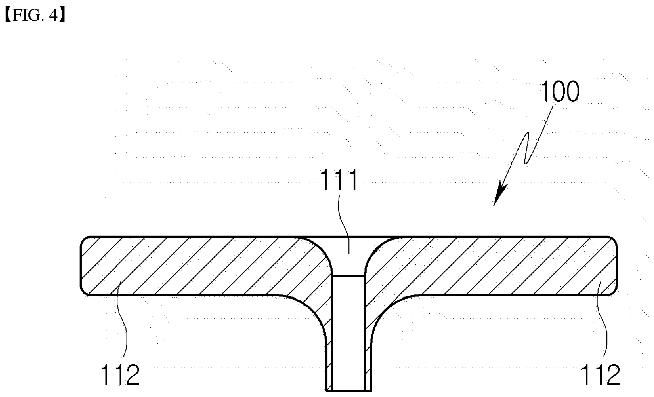

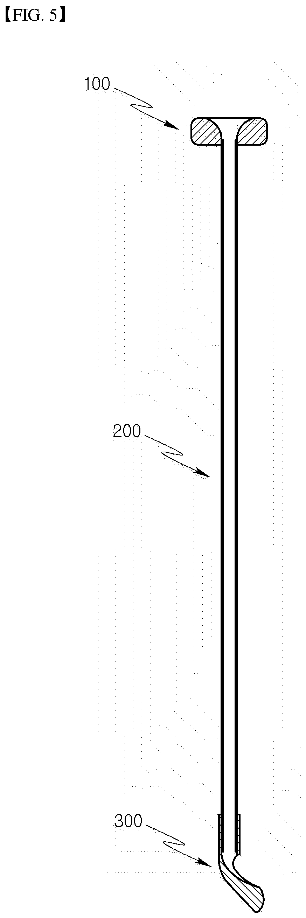

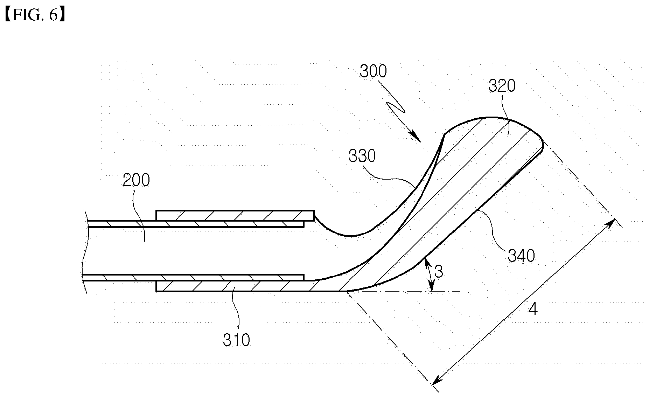

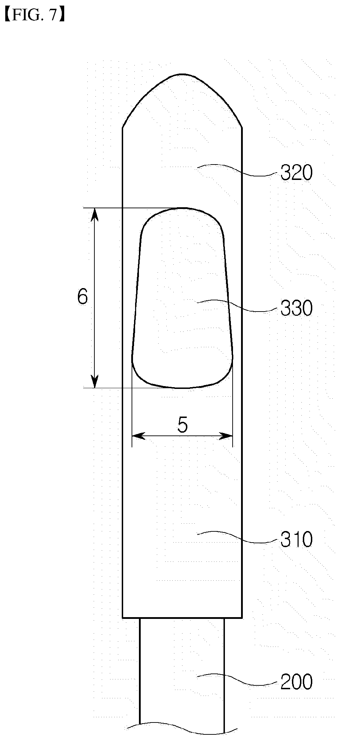

FIG. 1 is a perspective view of a drainage tube guide device according to an embodiment. FIG. 2 is a front view of a drainage tube guide device according to an embodiment. FIG. 3 is a side view of a drainage tube guide device according to an embodiment. FIGS. 4 and 5 are drawings for describing a head part of a drainage tube guide device according to an embodiment. FIGS. 6 and 7 are drawings for describing a peeling part of a drainage tube guide device according to another embodiment.

Referring to FIGS. 1 to 5, a drainage tube guide device according to an embodiment comprises a head part 100, a guide tube 200, and a peeling part 300.

In an embodiment, the head part 100 may be formed as a rod shape extending in both directions, and may include an inlet 111 through which a drainage tube is introduced from the upper surface.

In an embodiment, the inlet 111 may be formed to communicate with a hollow of a guide tube 200.

In an embodiment, the handle portion 112 of the head part 100 may be transformed into various shapes. As shown in the drawing, the handle portion may have a spherical shape formed by a rod or curved surface extending in both lateral directions, or may be one of a polyhedron having six or more sides.

In an embodiment, the handle portion 112 may further include a plurality of grip grooves (not shown) which are directed inward and spaced apart from each other for the user's grip as a rod or bar shape extending in both lateral directions so that it can function as a handle.

The grip grooves (not shown) may be formed as a plurality of grooves directed inward to fit the shape of the fingers for a comfortable grip when the user holds the handle portion 112 in his or her hand.

In an embodiment, the head part 100 may include an identification portion 113 which protrudes forward. The protrusion direction of the identification portion 113 and the protrusion direction of the peeling part 300 may be matched, thereby preventing the peeling part 300 from being introduced in the opposite direction to the direction of progression during abdominal introduction.

In an embodiment, the head part 100 may be welded to one end 1 of the guide tube 200.

The abdominal cavity is composed of the abdominal wall. The abdominal wall is largely composed of skin, subcutaneous fat tissue plane, muscle plane surrounded by fascia in the front and back, fascia, preperitoneal fat tissue plane, and peritoneum.

In an embodiment, the guide tube 200 may be introduced along the abdominal wall.

In an embodiment, the guide tube 200 may be formed as a hollow shape which communicates with the inlet 111, and may guide a drainage tube introduced through the inlet 111.

In an embodiment, the guide tube 200 may be extended downwardly on the lower surface of the head part 100.

In an embodiment, the guide tube 200 may be extended downwardly to have a straight shape as illustrated in the drawing.

The longitudinal axis of the abdominal cavity is usually about 30 to 50 cm, the transverse axis is about 20 to 30 cm, and the cross-sectional radius of the abdominal cavity is about 10 to 20 cm, which may vary depending on the body type.

The longest axis of the abdominal cavity is approximately 30 to 50 cm from the diaphragm above the liver to the end of the rectum (levator ani muscle), depending on the size of the adult body.

Considering the width and depth of the abdominal cavity of all adults, the guide tube 200 may have a length of 20 to 40 cm, in an embodiment.

In an embodiment, the guide tube 200 may be made of a plastic material having a hardness which does not bend, or a metallic material.

In an embodiment, the guide tube 200 may be made of a soft plastic material which is capable of bending.

In an embodiment, the peeling part 300 may be connected to the hollow of the guide tube 200, and may be introduced while peeling the tissue from the front. When the target point is reached, the drainage tube may be inserted through the inlet 111 of the head part 100.

In an embodiment, the peeling part 300 may be welded to the other end 2 of the guide tube 200.

Referring to FIGS. 6 and 7, the peeling part 300 may include, in an embodiment, a fixing portion 310, a protrusion portion 320, an extraction port 330, and an inclined portion 340.

In an embodiment, the fixing portion 310 may be coupled to the end of the guide tube 200.

In an embodiment, the fixing portion 310 may include a hollow formed therein, and the end of the guide tube 200 may be inserted into the hollow of the fixing portion 310 and welded.

In an embodiment, the protrusion portion 320 may be formed to protrude in an insertion direction.

In an embodiment, the protrusion portion 320 may be formed to be inclined downward in an end direction. That is, the protrusion portion 320 may have a round shape with a non-sharp end.

In an embodiment, the protrusion portion 320 may be formed in a wedge shape with a non-sharp end.

Through the protrusion portion 320 which has a non-sharp end, the target point may be reached while peeling the extraperitoneal fat tissue plane without damaging the tissue and organs.

In an embodiment, the extraction port 330 is connected to the hollow of the guide tube 200, and the drainage tube inserted into the guide tube 200 may be extracted.

In an embodiment, the extraction port 330 may have a shape of either a circle or an oval. The extraction port 330 may have various shapes other than a circle or an oval in an embodiment.

When the extraction port 330 has an oval shape, two drainage tubes are extracted, thereby shortening the surgical time and improving the surgical efficiency.

In an embodiment, the length 6 (e.g., 12.5 mm) of the extraction port 330 may be longer than the width 5 (e.g., 6 mm).

In an embodiment, the inclined portion 340 may be formed to extend upwardly from the fixing portion 310.

In an embodiment, the inclined portion 340 may be curved at an angle 3 of 25 to 55 degrees or less from the fixing portion 310.

In an embodiment, the inclined portion 340 may have a length 4 of 1 to 5 cm.

In the case of a curved mechanism, there is an advantage in that it is suitable for moving along the curved surface of the abdominal cavity, but when changing direction to reach the target point, the preperitoneal fat tissue plane may be excessively peeled, which may cause unexpected bleeding. In addition, since users are less familiar with using a curved-shaped mechanism than a straight-shaped mechanism, it may be difficult to use.

In contrast, in the case of the present invention, by using the straight-shaped guide tube 200, the peeling part 300 formed to protrude and slope upward in the direction of progression may minimize the peeling of the preperitoneal fat tissue plane when moving to the target point in the abdominal cavity, and can prevent bleeding due to excessive peeling.

The guide tube 200 of the present invention may have a length of 20 to 40 cm, and the inclined portion 340 may have a length of 1 to 5 cm and a curved shape with the angle 3 of 25 to 55 degrees or less. This numerical limitation is a range that can vary depending on the difference in body shape in order to reach the farthest target point from the abdominal drainage tube insertion site where the drainage tube is commonly inserted. Extraperitoneal drainage tube insertion minimizes movement of the drainage tube due to peristalsis of the intestines, thereby effectively draining body fluids in the abdominal cavity for a long period of time. Accordingly, it may increase the therapeutic effect compared to the existing method, that is, the method for inserting and fixing an intraperitoneal drainage tube through the abdominal cavity.

The present invention relates to a drainage tube guide device including the non-sharp protrusion portion 320, which can reach the target point while peeling the extraperitoneal fat tissue plane without damaging tissues and organs. Accordingly, since the drainage tube can be inserted through the retroperitoneum to drain body fluid within the abdominal cavity, the process of inserting and fixing the drainage tube into the abdominal cavity through an existing surgical site can be performed more easily, thereby enhancing the therapeutic effect.

Although the embodiments of the present invention have been described in detail above, the scope of the present invention is not limited thereto, and various modifications and improvements made by those skilled in the art using the basic concept of the present invention defined in the following claims also fall within the scope of the present invention.

Those skilled in the art related to the present embodiment will understand that the present invention can be implemented in a modified form without departing from the essential characteristics of the above-described description. Therefore, the disclosed methods should be considered from an illustrative rather than a restrictive perspective. The scope of the present invention is indicated by the claims, not the above description, and all differences within the equivalent scope should be interpreted as being included in the present invention.

## Claims

1. A drainage tube guide device comprising:
a head part which extends in one direction;
a guide tube which has a hollow formed therein and extends downward from the head part; and
a peeling part which is in communication with the hollow of the guide tube and peels tissue from a front while being inserted thereinto.

2. The drainage tube guide of claim 1, wherein the head part includes an inlet which communicates with the hollow of the guide tube so that a drainage tube is able to be inserted into the guide tube from an upper surface.

3. The drainage tube guide of claim 1, wherein the head part includes an identification portion protruding forward.

4. The drainage tube guide of claim 1, wherein the head part is any one of a bar shape extending laterally, a sphere forming a curved surface, and a polyhedron forming a polygon.

5. The drainage tube guide of claim 1, wherein a length of the guide tube is 20 cm or more to 40 cm or less.

6. The drainage tube guide of claim 1, wherein the guide tube has a straight shape.

7. The drainage tube guide of claim 1, wherein the peeling part includes:
a fixing portion which is connected to an end of the guide tube, and
a protrusion portion which is formed to protrude in an insertion direction.

8. The drainage tube guide of claim 7, wherein the fixing portion includes a hollow formed therein, and an end of the guide tube is inserted into the hollow of the fixing portion.

9. The drainage tube guide of claim 7, wherein the protrusion portion has a round shape with a non-sharp end.

10. The drainage tube guide of claim 7, wherein the peeling part further includes an extraction port which is connected to the hollow of the guide tube and through which a drainage tube inserted into the guide tube is extracted.

11. The drainage tube guide of claim 10, wherein the extraction port has a shape of either a circle or an oval.

12. The drainage tube guide of claim 7, wherein the peeling part further includes an inclined portion extending upwardly from the fixing portion.

13. The drainage tube guide of claim 12, wherein the inclined portion is curved at an angle of 25 degrees or more to 55 degrees or less from the fixing portion.

14. The drainage tube guide of claim 12, wherein a length of the inclined portion is 1 cm or more to 5 cm or less.
